# EUROPEAN PATENT SPECIFICATION

(11) **EP 4 435 048 B1**
(45) Date of publication and mention of the grant of the patent: **23.09.2026**
(21) Application number: 22898647.7
(22) Date of filing: 25.11.2022
(51) Int. Cl.: C09K 11/06, C09K 11/02, C08F 2/22, C08F 212/08, G01N 33/58, C08F 230/08, G01N 33/533

(54) **PARTICLES FOR SPECIMEN EXAMINATIONS**
PARTIKEL FÜR PROBENUNTERSUCHUNGEN
PARTICULES POUR EXAMENS D'ÉCHANTILLON

(30) Priority: 26.11.2021 JP 2021192077; 21.11.2022 JP 2022185946
(43) Date of publication of application: 25.09.2024
(73) Proprietor: CANON KABUSHIKI KAISHA, Tokyo 146-8501 (JP)
(72) Inventor: SAKAKIBARA Teigo, Tokyo 146-8501 (JP); KAKEGAWA Norishige, Tokyo 146-8501 (JP); NAKAJIMA Ikuo, Tokyo 146-8501 (JP); YAMAUCHI Fumio, Tokyo 146-8501 (JP); KANAZAKI Kengo, Tokyo 146-8501 (JP); MASUMURA Takahiro, Tokyo 146-8501 (JP); NAKAMURA Tomohiro, Tokyo 146-8501 (JP)
(74) Representative: WESER & Kollegen Patentanwälte PartmbB
(86) International application number: PCT/JP2022/043512
(87) International publication number: WO 2023/095865

(56) References cited:
- WO-A1-2010/029739
- WO-A1-2022/209952
- CN-A- 103 172 611
- CN-A- 103 172 941
- CN-A- 106 124 771
- JP-A- 2005 171 097
- JP-A- 2007 512 542
- JP-A- 2019 189 825
- JP-A- 2020 180 182
- JP-A- 2021 036 230
- JP-A- 2021 063 225
- KR-A- 20160 142 976
- KR-A- 20180 106 684
- KR-B1- 101 423 589
- AIKAWA TATSUO; MIZUNO AKIHIRO; KOHRI MICHINARI; TANIGUCHI TATSUO; KISHIKAWA KEIKI; NAKAHIRA TAKAYUKI: "Polystyrene latex particles containing europium complexes prepared by miniemulsion polymerization using bovine serum albumin as a surfactant for biochemical diagnosis", COLLOIDS AND SURFACES B: BIOINTERFACES, ELSEVIER AMSTERDAM, NL, vol. 145, 3 May 2016 (2016-05-03), NL , pages 152 - 159, XP029640038, ISSN: 0927-7765, DOI: 10.1016/j.colsurfb.2016.04.055

## Description

### [Technical Field]

The present invention relates to a particle for a specimen test and a method of producing the same.

### [Background Art]

In the fields of medicine and clinical tests, high-sensitivity detection or quantification of a trace amount of a biological component from, for example, blood or a collected part of an organ is required for investigating, for example, the cause and presence or absence of a disease. Among test techniques for biological components, immunoassays are widely utilized. In many of the immunoassays, a washing step called bound/free (B/F) separation is required. As an immunoassay that does not require the B/F separation, there is known a latex agglutination method utilizing an antigen-antibody reaction. In the latex agglutination method, latex particles each having supported thereon, for example, an antibody that specifically binds to a target substance are mixed with a liquid that may contain the target substance, and the degree of agglutination of the latex particles is measured.

In the latex agglutination method, the target substance is captured by the antibody bound to the latex particles and specific to the target substance, and a plurality of the latex particles are crosslinked via the captured target substance, with the result that the agglutination of the latex particles occurs. That is, the amount of the target substance in a liquid sample such as a biological sample can be quantified by evaluating the degree of the agglutination of the latex particles. The degree of the agglutination can be quantified by measuring and evaluating a change in amount of light transmitted through or scattered by the liquid sample.

The latex agglutination method can detect/quantitatively evaluate an antigen as the target substance in a simple and rapid manner, but has involved a problem with detection limits in that the antigen cannot be detected when its amount in the liquid sample such as the biological sample is small.

In order to improve detection sensitivity for the target substance, it is required that the degree of the agglutination be measured with higher sensitivity. That is, it is conceivable to replace a system for measuring the change in amount of the light transmitted through or scattered by the liquid sample with a method for detection/quantification utilizing a luminescence characteristic with higher sensitivity. Specifically, there has been proposed, for example, a specimen test method utilizing a fluorescence depolarization method (Patent Literatures 1 and 2).

In Patent Literature 1, it is proposed that an apparatus for the fluorescence depolarization method be improved to be clinically used.

In the fluorescence depolarization method, the B/F separation required in a general fluorescence measurement method is not required. Accordingly, use of the fluorescence depolarization method enables a simple specimen test as with the latex agglutination method. Further, it is conceived that use of the fluorescence depolarization method enables measurement by the same test system as that in the latex agglutination method by merely mixing a luminescent substance that specifically reacts with the measuring substance in a measurement process. Meanwhile, in Patent Literature 1, there is a proposal of use of a single molecule such as fluorescein as a luminescent material, which is applicable only to a drug, a low-molecular-weight antigen, and the like in principle.

Patent Literature 2 has solved the problem of Patent Literature 1, i.e., the problem in that the fluorescence depolarization method is applied only to a drug, a low-molecular-weight antigen, and the like. That is, in Patent Literature 2, with an aim to apply the fluorescence depolarization method to a macromolecule such as a protein, it is proposed to use, as a luminescent material, a material obtained by causing a dye having a long-lifetime luminescence characteristic to adsorb to latex particles. In Patent Literature 2, it is proposed that a high-molecular-weight substance be quantified by balancing a reduction in rotational Brownian motion of the substance in a liquid due to an increase in particle diameter and the length of emission lifetime based on the principle of the fluorescence depolarization method.

### [Citation List]

### [Patent Literature]

PTL 1: Japanese Patent Publication No. H3-52575
PTL 2: Japanese Patent No. 2893772

### [Summary of Invention]

### [Technical Problem]

However, in Patent Literature 2, a fluorescent substance is supported on the latex particles after synthesis of the particles, and hence an interaction between fluorescent substances adsorbed in the vicinity of surfaces of the particles or the like makes it difficult to stably determine the polarization anisotropy of the testing particles. Further, in Patent Literature 2, bovine serum albumin (BSA), which is a biomolecule, is supported on surfaces of the particles in order to suppress nonspecific adsorption, and hence there is a risk in that a lot-to-lot variation occurs owing to a broad particle size distribution and BSA, which is a protein.

Accordingly, also in Patent Literature 2, high-sensitivity detection of a target substance has been far from being achieved. Further relevant background art are CN106124771A and Aikawa Tatsuo, et al.: "Polystyrene latex particles containing europium complexes prepared by miniemulsion polymerization using bovine serum albumin as a surfactant for biochemical diagnosis",Colloids and Surfaces B: Biointerfaces, Elsevier Amsterdam, NL, vol. 145, 3 May 2016 (2016-05-03), pages 152-159, which disclose luminescent polystyrene latex particles prepared by miniemulsion polymerization and miniemulsion polymerization of styrene to produce luminescent polystyrene latex particles, respectively.

The present invention has been made in view of such background art, and an object of the present invention is to provide a particle that enables a high-sensitivity specimen test by a fluorescence depolarization method, and a method of producing the same.

### [Solution to Problem]

According to an embodiment of the present invention, there is provided a particle including a particle substrate,
wherein the particle substrate includes: a polymer containing a styrene unit and an organic silane unit; and a europium complex represented by the following formula (1), and
wherein the particle substrate contains a siloxane bond on at least a surface of the particle substrate:

   Eu(A)ₓ(B)_{y}(C)_{z} ··· (1)

   in the formula (1), (A) represents a ligand represented by the following formula (2), (B) represents a ligand represented by the following formula (3) or (4), and (C) represents a ligand represented by the following formula (5): 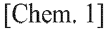 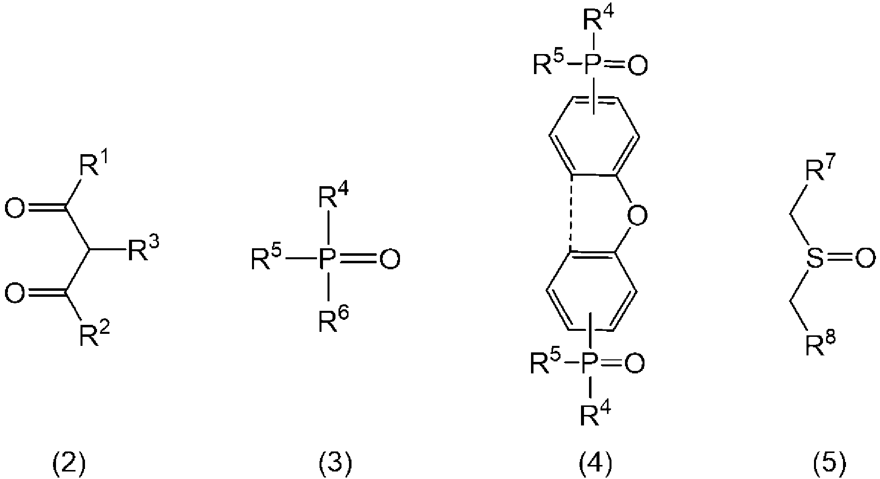
in the formulae (2) to (5), R¹ and R² each independently represent an alkyl group, a perfluoroalkyl group, a phenyl group, or a thiophene group, and the groups may each have a substituent, R³ represents a hydrogen atom or a methyl group, R⁴ and R⁵ each independently represent an alkyl group or a phenyl group, and the groups may each have a substituent, R⁶ represents an alkyl group, a phenyl group, or a triphenylene group, and the groups may each have a substituent, and R⁷ and R⁸ each independently represent an alkyl group or a phenyl group, and the groups may each have a substituent; in the formula (4), a bond represented by a broken line may or may not be present; the substituents are each independently any one of a methyl group, a fluoro group, a chloro group, or a bromo group; the alkyl groups each independently have 2 or more and 12 or less carbon atoms; and x, y, and z satisfy the following equations: $x = 3 ;$ $y = 1 or 2 ;$ $z = 0 or 1 ;$ and $x + y + z = 4 or 5 .$

In addition, the particle preferably further includes a layer containing a hydrophilic polymer.

Further features of the present invention will become apparent from the following description of exemplary embodiments with reference to the attached drawings.

### [Brief Description of Drawings]

[FIG. 1]

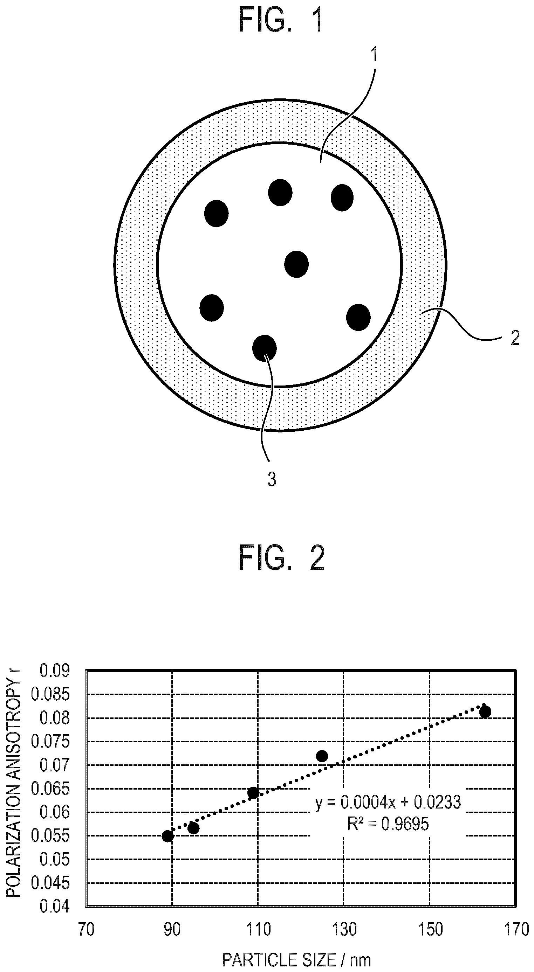

   FIG. 1 is a schematic view for illustrating the structure of a particle according to an embodiment of the present invention.
[FIG. 2]
   FIG. 2 shows the results of fluorescence polarization measurement of samples of Examples 1, 2, 3, 4, and 7, and is an explanatory graph of a relationship between values of polarization anisotropy <r> of the samples determined therefrom and their particle sizes.
[FIG. 3]

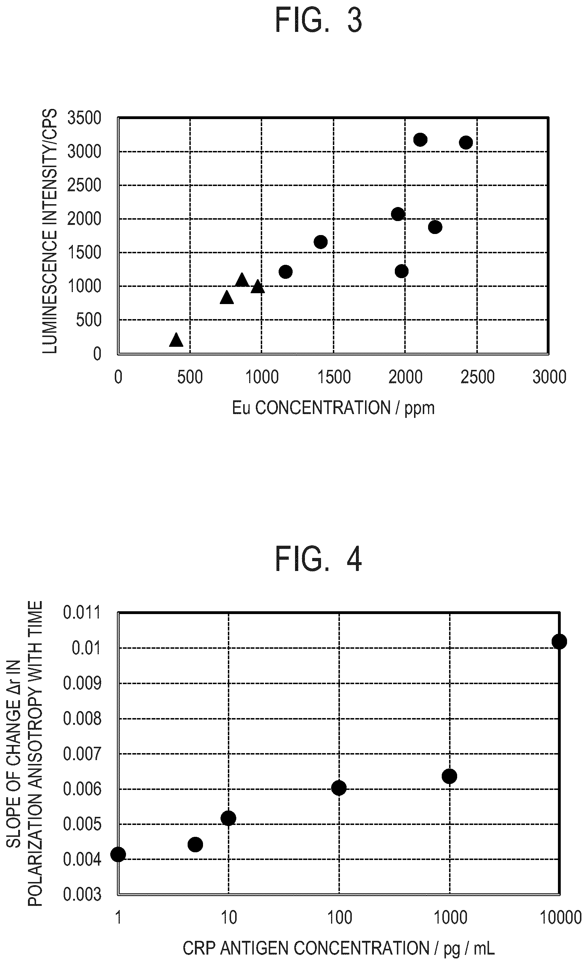

   FIG. 3 is an explanatory graph of a relationship between: luminescence intensities of samples of Examples 1, 2, 3, 4, 5, 6, and 7, and Comparative Examples 1, 2, 3, and 4; and their europium contents.
[FIG. 4]
   FIG. 4 shows the evaluation results of affinity particles 3, and is an explanatory graph of the results of quantification of CRP antigen concentrations through use of a fluorescence depolarization method.

### [Description of Embodiments]

Preferred embodiments of the present invention are described in detail below. However, the embodiments are not intended to limit the scope of the present invention.

An example of a particle according to this embodiment is described in detail with reference to FIG. 1.

As illustrated in FIG. 1, the particle according to this embodiment includes a particle substrate 1 containing a europium complex 3, and a hydrophilic layer 2 coating the surface thereof.

The particle according to this embodiment has a small particle size distribution, and the surface of the particle is coated with the hydrophilic layer.

The europium complex 3 is present inside the particle.

FIG. 1 is a schematic view for illustrating an example of the particle according to this embodiment. The diameter of the particle illustrated in FIG. 1 is 25 nm or more and 500 nm or less.

The diameter of each of the particles may be determined by a dynamic light scattering method. When particles dispersed in a solution are irradiated with laser light and the resultant scattered light is observed with a photon detector, an intensity distribution due to interference of the scattered light is constantly fluctuating because the particles are constantly shifting their positions by Brownian motion.

The dynamic light scattering method is a measurement method for observing the state of the Brownian motion as a fluctuation in scattered light intensity. The fluctuation of scattered light with respect to time is expressed as an autocorrelation function, and a translational diffusion coefficient is determined. A Stokes diameter is determined from the determined diffusion coefficient, and the size of each of the particles dispersed in the solution can be derived.

The particle according to this embodiment desirably has nothing provided on the surfaces of its particles from the viewpoint of keeping the uniformity and monodispersity of the particles. However, for the purpose of use in a specimen test, nonspecific adsorption of substances other than the target onto the particles needs to be prevented, and hence the particle needs to be coated for keeping its surface hydrophilic.

A method involving supporting BSA on the surface of each of the particles is widely used as a technique for keeping hydrophilicity, but this method may cause a lot variation. In view of this, the surface of the particle of this embodiment was coated with a hydrophilic polymer.

The particle according to this embodiment can emit phosphorescence having a long lifetime because the particle contains the europium complex. In the particle according to this embodiment, an average particle diameter that is the average of the diameters of the particles is preferably 25 nm or more and 500 nm or less, and the average particle diameter is more preferably 50 nm or more and 300 nm or less. When the average particle diameter is more than 500 nm, the polarization anisotropy before aggregation becomes higher, resulting in a small difference from the polarization anisotropy after the aggregation reaction. In addition, when the average particle diameter is less than 25 nm, a change between sizes before and after the aggregation becomes smaller to make it difficult to grasp the change of the polarization anisotropy through phosphorescent fluorescence depolarization.

By reducing the particle size distribution of the particles and introducing the europium complex indicating polarized luminescence into the particles, a change in polarized luminescence characteristic can be grasped even when the dispersion state of the particles in the liquid undergoes a slight change. Specifically, even if the concentration of the target substance in the solution is from about a nanogram to about a picogram per mL, when the particles aggregate via the target substance, a change in rotational Brownian motion of the particles can be grasped as a change in polarization anisotropy.

The "polarized luminescence" refers to the following phenomenon: when a luminescent dye having anisotropy in transition moment (transition dipole moment) uses polarized light along its transition moment as excitation light, its luminescence is also polarized light along the transition moment. The europium complex shows fluorescent luminescence based on energy transfer from the ligand to the central metal ion, and hence the transition moment is complicated, but red luminescence around 610 nm, which is derived from electronic transition from the lowest excited state 5D0 to 7F2, is emitted as polarized light.

The principle of fluorescence depolarization is the measurement of a shift in transition moment due to the rotational motion of a luminescent material during the occurrence of polarized luminescence. The rotational motion of the luminescent material may be represented by the equation (6): $Q = 3 Vη / kT$ where Q represents the rotational relaxation time of the material, V represents the volume of the material, η represents the viscosity of a solvent, "k" represents the Boltzmann constant, and T represents an absolute temperature.

The rotational relaxation time of the material is a period of time required for a molecule to rotate by an angle θ (68.5°) at which cosθ=1/e.

It is found from the equation (6) that the rotational relaxation time of the luminescent material is proportional to the volume of the material, that is, when the luminescent material has a particulate shape, the cube of the particle diameter. Meanwhile, a relationship between the emission lifetime and degree of polarization of the luminescent material may be represented by the equation (7): $p 0 / p = 1 + A \left(τ/Q\right)$ where p0 represents a degree of polarization at a time when the material is stationary (Q=∞), "p" represents the degree of polarization, A is a constant, *τ* represents the emission lifetime of the material, and Q represents the rotational relaxation time.

It is found from the equation (6) and the equation (7) that the degree of polarization is influenced by the emission lifetime of the luminescent material and the rotational relaxation time, that is, the volume of the luminescent material, and the volume depends on the particle diameter, and hence, in other words, the degree of polarization is influenced by the balance between the particle diameter and emission lifetime of the luminescent material.

When the degree of polarization of the luminescent material represented by the equation (7) is determined experimentally, it is appropriate that polarized light be allowed to enter the luminescent material, and luminescence be detected in a 90° direction with respect to the traveling direction and vibration direction of excitation light. In this case, it is appropriate that the detected light be detected by being divided into polarized light components in parallel and perpendicular directions with respect to the polarized light that is the incident light, and polarization anisotropy be evaluated by a numerical expression represented by the following equation (8): $r \left(t\right) = \left(I//\left(t\right)-GI⊥\left(t\right)\right) / \left(I//\left(t\right)-2GI⊥\left(t\right)\right)$ where r(t) represents polarization anisotropy at a time "t", I//(t) represents the luminescence intensity of a luminescence component parallel to the excitation light at the time "t", I⊥(t) represents the luminescence intensity of a luminescence component perpendicular to the excitation light at the time "t", and G represents a correction value, that is, the ratio of I⊥/I// measured with excitation light having a vibration direction different by 90° from that of the excitation light used for sample measurement.

That is, when the particle size and the emission lifetime fall within appropriate ranges, a change in size of the luminescent material due to, for example, a reaction with the target substance can be sensitively read as a change in polarization anisotropy. That is, the r(t) of the unaggregated luminescent material is observed to be low, and the r(t) of the aggregated luminescent material is observed to be high. This is the principle of a fluorescence depolarization method.

The polarization anisotropy refers to a value for the degree of polarization corrected with G and 2G, and the degree of polarization is a value obtained by removing G and 2G from the equation (8). In actual measurement, the correction value G is required, and hence the polarization anisotropy is determined.

It is preferred that the particle according to the embodiment of the present invention have a polarization anisotropy <r> of 0.01 or more, which is determined by the following equation (9).
[Math. 1] $\begin{matrix}\left〈r\right〉 = \frac{{I}_{VV} - G ∗ {I}_{VH}}{{I}_{VV} + 2 ∗ G ∗ {I}_{VH}} \\ G = \frac{{I}_{HV}}{{I}_{HH}}\end{matrix}$ in the equation (9),
<r> represents the polarization anisotropy,
Ivv represents the luminescence intensity of a luminescence component having a vibration direction parallel to that of a first polarized light beam at the time of excitation by the first polarized light beam,
I_{VH} represents the luminescence intensity of a luminescence component having a vibration direction orthogonal to that of the first polarized light beam at the time of excitation by the first polarized light beam,
I_{HV} represents the luminescence intensity of a luminescence component having a vibration direction orthogonal to that of a second polarized light beam having a vibration direction orthogonal to that of the first polarized light beam at the time of excitation by the second polarized light beam,
I_{HH} represents the luminescence intensity of a luminescence component having a vibration direction parallel to that of the second polarized light beam having a vibration direction orthogonal to that of the first polarized light beam at the time of excitation by the second polarized light beam, and
G represents a correction value.

### (Particle Substrate 1)

FIG. 1 is a view for illustrating an example of a spherical particle, and the particle includes the particle substrate 1. An example in which both the particle and the particle substrate 1 are in spherical shapes is illustrated in FIG. 1, but the shapes of the particle of this embodiment and the particle substrate 1 are not limited thereto. The particle substrate 1 is not particularly specified as long as the particle substrate 1 is a material capable of stably incorporating the europium complex, but the substrate is preferably a polymer containing a styrene unit and an organic silane unit. In particular, for example, a polymer obtained by polymerizing a composition containing styrene as a main component and a radically polymerizable organic silane is suitably used. When the composition contains styrene as the main component, particles having a uniform particle size distribution can be produced by an emulsion polymerization method to be described later. In addition, when a polymer containing an organic silane unit is adopted, a silanol group (Si-OH) is produced in the polymer in an aqueous medium, and particle substrate surfaces form a siloxane bond (Si-O-Si) to each other, via which the hydrophilic layer to be described later or a ligand can be provided. The particle according to this embodiment preferably has a ligand-bonding functional group capable of bonding a ligand to the outside of the particle substrate.

### (Hydrophilic Layer 2)

The hydrophilic layer 2 is formed by incorporating a hydrophilic polymer or a hydrophilic molecule on the outside of the particle substrate 1. The hydrophilic polymer or the hydrophilic molecule is a polymer or molecule containing a hydrophilic group, and specific examples of the hydrophilic group include molecules or polymers each having a hydroxy group, an ether, pyrrolidone, or a betaine structure. Specific examples of the hydrophilic polymer include polyethylene glycol, polyvinylpyrrolidone, a polymer of sulfobetaine, a polymer of phosphobetaine, and polyglycidyl methacrylate whose molecule has an end modified with a hydroxy group by ring-opening a glycidyl group, and those hydrophilic polymers may each be used as a main component of the hydrophilic layer 2. Alternatively, the hydrophilic layer 2 may be formed by directly providing a single molecule having a hydrophilic group on the surface of the particle substrate 1 through use of a silane coupling agent or the like. The thickness of the hydrophilic layer 2 is not limited, but does not need to be set to be large beyond a thickness with which hydrophilicity can be exhibited. When the hydrophilic layer 2 is excessively thick, there is a risk in that the hydrophilic layer becomes hydrogel-like and is hydrated by the influence of ions in the solvent, to thereby make its thickness unstable. The thickness of the hydrophilic layer 2 is suitably 1 nm or more and 15 nm or less.

### (Europium Complex 3)

The particle of this embodiment includes the europium complex 3 as a luminescent dye. The europium complex 3 has a feature in that the wavelength and intensity of its luminescence are hardly influenced by the surroundings, and hence the luminescence has a long lifetime. The europium complex 3 includes a europium element and a ligand. In consideration of the emission lifetime, a visible emission wavelength region, and the like, the luminescent dye needs to be a europium complex. Europium generally has an emission lifetime of from 0.1 ms to 1.0 ms. The emission lifetime and the rotational relaxation time obtained from the equation (6) need to be appropriately adjusted. In the case of europium in a water dispersion, when the diameter of the particle is from about 50 nm to about 300 nm, the polarization anisotropy represented by the equation (8) significantly changes before and after aggregation.

At least one of the constituent ligands of the europium complex 3 is a ligand having a light-collecting function. The "light-collecting function" refers to an action of being excited at a particular wavelength to excite the central metal of the complex through energy transfer. In addition, it is preferred that the constituent ligands of the europium complex 3 include a ligand such as a β-diketone to prevent coordination of a water molecule. The ligand such as the β-diketone coordinated to a europium ion suppresses a deactivation process due to the transfer of energy to a solvent molecule or the like to provide strong fluorescent luminescence.

The europium complex 3 may be a polynuclear complex.

The europium complex 3 is preferably represented by the formula (1):

Eu(A)ₓ(B)_{y}(C)_{z} ··· (1)

in the formula (1), (A) represents a ligand represented by the following formula (2), (B) represents a ligand represented by the following formula (3) or (4), and (C) represents a ligand represented by the following formula (5): 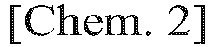 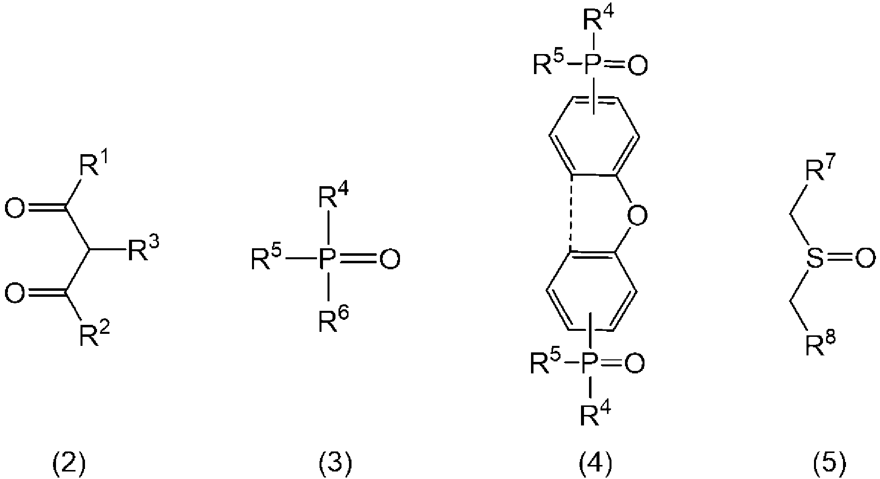 in the formulae (2) to (5), R¹ and R² each independently represent an alkyl group, a perfluoroalkyl group, a phenyl group, or a thiophene group, and the groups may each have a substituent, R³ represents a hydrogen atom or a methyl group, R⁴ and R⁵ each independently represent an alkyl group or a phenyl group, and the groups may each have a substituent, R⁶ represents an alkyl group, a phenyl group, or a triphenylene group, and the groups may each have a substituent, and R⁷ and R⁸ each independently represent an alkyl group or a phenyl group, and the groups may each have a substituent; a bond represented by a broken line in the formula (4) may or may not be present; the substituents are each independently any one of a methyl group, a fluoro group, a chloro group, or a bromo group; the alkyl groups each independently have 2 or more and 12 or less carbon atoms; and x, y, and z satisfy the following equations: $x = 3 ;$ $y = 1 or 2 ;$ $z = 0 or 1 ;$ and $x + y + z = 4 or 5 .$

A preferred specific example of the ligand represented by the formula (2) is 2-thenoyltrifluoroacetone.

A preferred specific example of the ligand represented by the formula (3) is triphenylphosphine oxide.

A preferred specific example of the ligand represented by the formula (5) may be dibenzyl sulfoxide.

Preferred specific examples of the ligand represented by the formula (4) may include ligands represented by the following formulae (10) and (11). 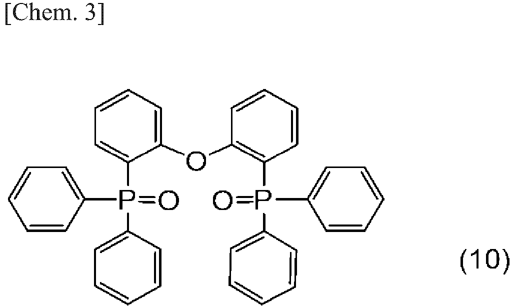 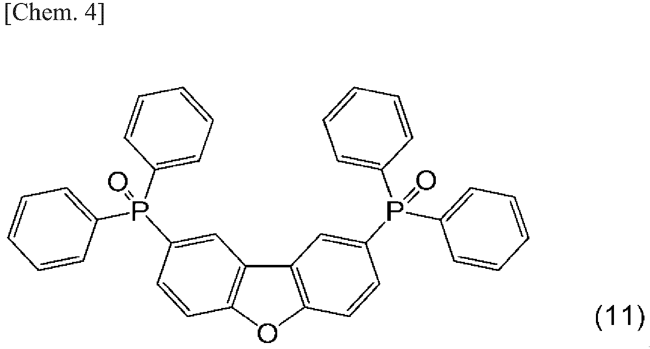 In addition, specific examples of the europium complex include [tris(2-thenoyltrifluoroacetone)(bis(triphenylphosphineoxide))europium(III)], [tris(2-thenoyltrifluoroacetone)(triphenylphosphineoxide)(dibenzylsulfoxide)europium(II I)], and [tris(2-thenoyltrifluoroacetone)(phenanthroline)europium(III)].

At the time of a state in which the Brownian rotational motion of the europium complex 3 can be regarded as stationary in a medium, the polarization anisotropy represented by the equation (8) is desirably 0.08 or more. The state in which the Brownian rotational motion can be regarded as stationary refers to a state in which the rotational relaxation time of the particle is sufficiently longer than the emission lifetime of the europium complex 3.

The europium complex 3 is preferably incorporated in as large an amount as possible into the particle substrate 1 because a luminescence intensity per particle becomes stronger. Meanwhile, when the molecules of the europium complex 3 aggregate in the particle substrate 1, an interaction between ligands influences the excitation efficiency of the europium complex 3 and the like to make it difficult to measure the polarization anisotropy while keeping reproducibility. Whether the europium complex 3 shows non-aggregated luminescence behavior in the particle substrate 1 may be judged from an excitation spectrum of the sample.

Particles having strong luminescence not only enable high-sensitivity measurement, but also enable an increase in biochemical reaction rate because luminescence is kept even when their particle diameters are reduced. As the particle diameters become smaller, the diffusion coefficient of Brownian motion in the liquid becomes larger, and hence the reaction can be detected in a shorter period of time.

When a liquid having the particle according to this embodiment dispersed therein is used, a change in anisotropy of polarized luminescence can be detected with high sensitivity in correspondence to the aggregation/dispersion behavior of the particle. Accordingly, a dispersion obtained by dispersing the particle according to this embodiment in an aqueous medium can be utilized as a high-sensitivity test reagent through use of a fluorescence depolarization method. A buffer solution may be used as the aqueous medium. In addition, a surfactant, a preservative, a sensitizer, or the like may be added into the aqueous medium in order to enhance the stability of the liquid having the particle according to this embodiment dispersed therein.

### (Method of producing Particle)

Next, an example of a method of producing the particle according to this embodiment is described.

The method of producing the particle according to this embodiment includes a step (first step) of mixing radically polymerizable monomers including at least styrene and a radically polymerizable organic silane, a radical polymerization initiator, a polarized luminescent europium complex, and a hydrophilic polymer with an aqueous medium to prepare an emulsion.

Further, the method of producing the particle according to this embodiment includes a step (second step) of heating the emulsion to polymerize the radically polymerizable monomers.

The particle according to this embodiment is a particle obtained by copolymerizing compounds each having a double bond having radical polymerizability.

Further, the method of producing the particle according to this embodiment may include a step (third step) of providing a ligand-bonding functional group to be described later on the surface of the particle. Herein, the ligand-bonding functional group refers to a functional group that can bond a ligand. Specifically, any one of a carboxy group, an amino group, a thiol group, an epoxy group, a maleimide group, a succinimidyl group, or an alkoxysilyl group (silicon alkoxide structure) may be used.

### (Radically Polymerizable Monomers)

The production of the particle is performed by polymerizing the radically polymerizable monomers, and the radically polymerizable monomers include at least styrene and the radically polymerizable organic silane. The radically polymerizable monomers may further include a monomer selected from the group consisting of: an acrylate-based monomer; and a methacrylate-based monomer. Examples of the monomers to be used in the production of the particle may include butadiene, vinyl acetate, vinyl chloride, acrylonitrile, methyl methacrylate, methacrylonitrile, methyl acrylate, and mixtures thereof. That is, one kind or a plurality of kinds of those monomers may be used in addition to styrene and the radically polymerizable organic silane. In addition, a monomer having two or more double bonds per molecule such as divinylbenzene may be used as a crosslinking agent.

The inclusion of the radically polymerizable organic silane in the radically polymerizable monomers provides a siloxane bond on the particle substrate 1. Examples of the radically polymerizable organic silane may include vinyltrimethoxysilane, vinyltriethoxysilane, p-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, 3-acryloxypropyltrimethoxysilane, and combinations thereof. The use of the radically polymerizable organic silane serves to form a backbone of an inorganic oxide in the particle substrate 1 to improve the physical and chemical stability of the particle. Further, the use of the radically polymerizable organic silane enhances affinity between the particle substrate 1 and each of the hydrophilic polymer and the ligand-bonding functional group.

Further, the inclusion of the radically polymerizable organic silane in the radically polymerizable monomers provides a silanol group on the surface of the particle substrate 1. The silanol group and the hydrophilic polymer such as PVP form a hydrogen bond. Thus, the hydrophilic polymer such as PVP more strongly adsorbs to the surface of the particle substrate 1.

### (Radical Polymerization Initiator)

A wide range of compounds selected from, for example, azo compounds and organic peroxides may each be used as the radical polymerization initiator. Specific examples thereof may include 2,2'-azobis(isobutyronitrile), 2,2'-azobis(2,4-dimethylvaleronitrile), 2,2'-azobis(2-methylbutyronitrile), 4,4'-azobis(4-cyanovaleric acid), 2,2'-azobis(2-methylpropionamidine) dihydrochloride, dimethyl 2,2'-azobis(2-methylpropionate), tert-butyl hydroperoxide, benzoyl peroxide, ammonium persulfate (APS), sodium persulfate (NPS), and potassium persulfate (KPS).

### (Hydrophilic Polymer)

The particle may include the hydrophilic polymer as the hydrophilic layer 2. The hydrophilic polymer preferably suppresses nonspecific adsorption. Examples of the hydrophilic polymer include hydrophilic polymers each containing a unit having an ether, a betaine, or a pyrrolidone ring. It is preferred that the layer containing the hydrophilic polymer be included in the synthesized particle, and be mainly present on the particle surface on the outside of the particle substrate. Herein, a polymer having a pyrrolidone ring is sometimes abbreviated as "PVP". When the PVP is fed at the time of the synthesis of the particle, a nonspecific adsorption-suppressing ability and a ligand-bonding ability can be simultaneously imparted to the particle. The PVP to be fed at the time of the synthesis has higher hydrophilicity than that of each of the radically polymerizable monomers, and hence is present at an interface between the solvent and the particle substrate that is being polymerized at the time of the synthesis. The particle substrate adsorbs the PVP onto the outside thereof by involving part of the PVP at the time of the polymerization, or by physical/chemical adsorption such as an interaction between a pyrrolidone ring and styrene (radically polymerizable monomer).

The molecular weight of the PVP is preferably 10,000 or more and 100,000 or less, more preferably 40,000 or more and 70,000 or less. When the molecular weight is less than 10,000, the hydrophilicity of the surface of the particle is weak, and hence nonspecific adsorption is liable to occur. When the molecular weight is more than 100,000, the hydrophilic layer becomes so thick as to gel, thereby becoming difficult to handle.

In addition to the PVP, another hydrophilic polymer may be added as a protective colloid at the time of the synthesis of the particle.

In addition, the particle satisfies A2-A1≤0.1.

A1 and A2 are defined as described below. That is, with regard to a mixture obtained by adding 30 µL of a 0.1 wt% dispersion of the particle to 60 µL of a buffer solution mixed with 16 µL of human serum diluted 15-fold, the absorbance of the mixture immediately after the addition is represented by A1, and the absorbance of the mixture after being left to stand at 37°C for 5 minutes after the addition is represented by A2. The absorbances are measured at an optical path of 10 mm and a wavelength of 572 nm.

A particle showing an A2-A1 of 0.1 or less has little nonspecific adsorption of impurities in serum, and hence is preferred.

### (Aqueous Medium)

The aqueous medium (aqueous solution) to be used for the above-mentioned method of producing the particle preferably contains water at 80 wt% or more and 100 wt% or less in the medium. The aqueous medium is preferably water or a water-soluble organic solvent, and examples thereof include solutions each obtained by mixing water with methanol, ethanol, isopropyl alcohol, or acetone. When an organic solvent other than water is incorporated at more than 20 wt%, dissolution of the polymerizable monomers may occur at the time of the production of the particles.

In addition, the aqueous medium preferably has its pH adjusted to 6 or more and 9 or less in advance. When the pH has a value of less than 6 or more than 9, there is a risk in that an alkoxide group or silanol group of the radically polymerizable organic silane undergoes condensation polymerization or a reaction with another functional group before the formation of the polymer, leading to aggregation of the particles to be obtained. In this embodiment, the alkoxide is not intentionally subjected, before the polymerization, to condensation polymerization.

The above-mentioned pH is preferably adjusted using a pH buffer, but may be adjusted with an acid or a base.

Other than the foregoing, a surfactant, an antifoaming agent, a salt, a thickener, or the like may be used by being added at a ratio of 10% or less with respect to the aqueous medium.

In the production of the particle according to this embodiment, it is preferred that, first, the PVP be dissolved in the aqueous medium whose pH has been adjusted to from 6 to 9. The content of the PVP is preferably 0.01 wt% or more and 10 wt% or less, more preferably from 0.03 wt% to 5 wt% with respect to the aqueous medium. When the content is less than 0.01 wt%, the amount of adsorption onto the particle substrate is small, and the effect thereof is not expressed. In addition, when the content is more than 10 wt%, there is a risk in that the viscosity of the aqueous medium is increased to preclude sufficient stirring.

Subsequently, the radically polymerizable monomers including the styrene (A) and the radically polymerizable organic silane (B) are added into the above-mentioned aqueous medium to prepare an emulsion. A weight ratio between the styrene (A) and the radically polymerizable organic silane (B) is from 6:4 to 100:1. Further, the prepared emulsion is mixed with the europium complex. At this time, when the solubility of the europium complex is low, a water-insoluble organic solvent may be added. A weight ratio between the europium complex and the radically polymerizable monomers is from 1:1,000 to 1:10.

When the weight ratio between the styrene (A) and the radically polymerizable organic silane (B) is less than 6:4, there is a risk in that the specific gravity of the particles as a whole is increased, resulting in remarkable sedimentation of the particles. In addition, in order to increase adhesiveness between the PVP and luminescent particles, it is desired that the weight ratio between the styrene (A) and the radically polymerizable organic silane (B) be set to 100:1 or more.

A weight ratio between the weight of the aqueous medium and the total amount of the radically polymerizable monomers is preferably from 5:5 to 9.9:0.1. When the weight ratio between the weight of the aqueous medium and the total amount of the radically polymerizable monomers is less than 5:5, remarkable aggregation of the particles to be produced may occur. In addition, when the weight ratio between the weight of the aqueous medium and the total amount of the radically polymerizable monomers is more than 9.9:0.1, although there is no problem with the production of the particles, the production amount thereof may be reduced.

The radical polymerization initiator is used by being dissolved in water, a buffer, or the like. The radical polymerization initiator may be used between 0.5 mass% and 10 mass% in the emulsion with respect to the total weight of the styrene (A) and the radically polymerizable organic silane (B).

In the above-mentioned step of heating the emulsion, it is only required that the entire emulsion be uniformly heated. A heating temperature may be arbitrarily set between 50°C and 80°C, and a heating time may be arbitrarily set between 2 hours and 24 hours. Through the heating of the emulsion, the radically polymerizable monomers are polymerized.

The particle of this embodiment may have a ligand-bonding functional group on its surface. The ligand-bonding functional group is not particularly limited as long as the functional group can bond an antibody, an antigen, an enzyme, or the like. However, for example, the functional group may be a carboxy group, an amino group, a thiol group, an epoxy group, a maleimide group, a succinimidyl group, or a silicon alkoxide group, or contain any of those functional groups. For example, a silane coupling agent having the ligand-bonding functional group and the synthesized particle may be mixed to provide the functional group on the particle surface. Specifically, an aqueous solution of a silane coupling agent having a carboxy group may be prepared and mixed with a dispersion of the synthesized particle to provide the carboxy group on the particle surface. At this time, a dispersant such as Tween 20 may be added to the reaction solution. A reaction temperature may be arbitrarily set between 0°C and 80°C, and a reaction time may be arbitrarily set between 1 hour and 24 hours. In order to suppress an abrupt condensation reaction of the silane coupling agent, it is suitable that the temperature be set to be equal to or lower than a room temperature of about 25°C, and the reaction time be set to from about 3 hours to about 14 hours. Depending on the ligand-bonding functional group, the reaction with the particle surface may be promoted by adding an acid or alkali catalyst.

The particle of this embodiment can be utilized as a particle for a specimen test by bonding a ligand such as any of various antibodies thereto. An optimal technique for bonding an antibody of interest or the like through utilization of a functional group present on the hydrophilic layer 2 only needs to be selected.

### (Ligand/Affinity Particle)

In this embodiment, there can be provided an affinity particle including the particle according to this embodiment and a ligand bonded to a ligand-bonding functional group.

The particle of this embodiment preferably includes a ligand specific to the target substance. By including the ligand, the particle of this embodiment enables detection/quantification of the target substance based on a fluorescence depolarization method. In this embodiment, the "ligand" refers to a compound that specifically binds to a particular target substance.

The ligand binds to the target substance at a predetermined site, and has selectively or specifically high affinity. Any substance that exhibits an interaction with some substance may serve as the target substance. Examples of the target substance may include an antigen, an antibody, a low-molecular-weight compound, various receptors, an enzyme, a substrate, a nucleic acid, a cytokine, a hormone, a neurotransmitter, a transmitter, and a membrane protein. Examples of the antigen include an allergen, a bacterium, a virus, a cell, a cell membrane constituent, a cancer marker, various disease markers, an antibody, a blood-derived substance, a food-derived substance, a natural product-derived substance, and any low-molecular-weight compound. Examples of the nucleic acid include DNA, RNA, or cDNA derived from a bacterium, a virus, or a cell, a part or fragment thereof, a synthetic nucleic acid, a primer, and a probe. Examples of the low-molecular-weight compound include a cytokine, a hormone, a neurotransmitter, a transmitter, a membrane protein, and a receptor therefor. Any compound that shows affinity for a particular substance may be used as the ligand. Examples of the ligand and the target substance, or a combination of the target substance and the ligand may include the following. That is, the examples may include: an antigen and an antibody; a low-molecular-weight compound and a receptor therefor; an enzyme and a substrate; and nucleic acids complementary to each other. Further, the examples may include an antibody and any of the following substances specific thereto: an allergen, a bacterium, a virus, a cell, a cell membrane constituent, a cancer marker, various disease markers, an antibody, a blood-derived substance, a food-derived substance, a natural product-derived substance, and any low-molecular-weight compound. Further, the examples may include a receptor and any of the following substances specific thereto: a low-molecular-weight compound, a cytokine, a hormone, a neurotransmitter, a transmitter, and a membrane protein. Further, the examples may include DNA, RNA, or cDNA derived from a bacterium, a virus, or a cell, a part or fragment thereof, a synthetic nucleic acid, a primer, or a probe, and a nucleic acid having complementarity thereto. Other than the foregoing, any combination known to have affinity may be used as the combination of the target substance and the ligand. A typical example of the ligand in this embodiment is any one of an antibody, an antigen, and a nucleic acid.

**In** this embodiment, a hitherto known method may be applied to a chemical reaction for chemically bonding the ligand-bonding functional group included in the particle according to this embodiment and the ligand to the extent that the object of this embodiment can be achieved. **In** addition, when the ligand is amide-bonded, a catalyst such as 1-[3-(dimethylaminopropyl)-3-ethylcarbodiimide] may be appropriately used.

The affinity particle in this embodiment may be preferably applied to a latex immunoagglutination measurement method utilized widely in the fields of clinical tests, biochemical research, and the like.

### (Test Reagent for In Vitro Diagnosis)

A test reagent for *in vitro* diagnosis, that is, a test reagent for use in the detection of a target substance in a specimen by *in vitro* diagnosis in this embodiment includes the affinity particle according to this embodiment and a dispersion medium for dispersing the affinity particle. The amount of the affinity particle according to this embodiment to be incorporated into the test reagent in this embodiment is preferably from 0.000001 mass% to 20 mass%, more preferably from 0.0001 mass% to 1 mass%. The test reagent according to this embodiment may include, in addition to the affinity particle according to this embodiment, a third substance, such as an additive or a blocking agent, to the extent that the object of this embodiment can be achieved. The reagent may include a combination of two or more kinds of third substances, such as an additive and a blocking agent. Examples of the dispersion medium to be used in this embodiment include various buffer solutions, such as a phosphate buffer solution, a glycine buffer solution, a Good's buffer solution, a Tris buffer solution, and an ammonia buffer solution, but the dispersion medium included in the test reagent in this embodiment is not limited thereto.

When the test reagent in this embodiment is used for the detection of an antigen or an antibody in a specimen, an antibody or an antigen may be used as the ligand.

### (Test Kit)

A test kit for use in the detection of a target substance in a specimen by *in vitro* diagnosis in this embodiment includes the above-mentioned reagent and a case enclosing the reagent. The kit according to this embodiment may contain a sensitizer for promoting the aggregation of the particles at the time of an antigen-antibody reaction. Examples of the sensitizer include polyvinyl alcohol, polyvinylpyrrolidone, and polyalginic acid, but are not limited thereto. In addition, the test kit according to this embodiment may include a positive control, a negative control, a serum diluent, or the like. As a medium for the positive control or the negative control, there may be used serum free of a measurable target substance, physiological saline, or a solvent. The test kit according to this embodiment may be used for the method of detecting a target substance according to this embodiment in the same manner as a related-art kit for use in detection of a target substance in a specimen by *in vitro* diagnosis. In addition, the concentration of the target substance may also be measured by a hitherto known method, and in particular, the test kit is suitably used for the detection of a target substance in a specimen by a latex agglutination method.

### (Detection Method)

A method of detecting a target substance in a specimen by *in vitro* diagnosis in this embodiment includes a step of mixing the affinity particle according to this embodiment with a specimen that may contain the target substance. In addition, the mixing of the affinity particle according to this embodiment with the specimen is preferably performed at a pH in the range of from 3.0 to 11.0. In addition, a mixing temperature falls within the range of from 20°C to 50°C, and a mixing time falls within the range of from 1 minute to 60 minutes. In addition, this detection method preferably uses a solvent. In addition, the concentration of the affinity particle according to this embodiment in the detection method according to this embodiment is preferably from 0.000001 mass% to 1 mass%, more preferably from 0.00001 mass% to 0.001 mass% in a reaction system. In the method of detecting a target substance in a specimen according to this embodiment, an aggregation reaction occurring as a result of the mixing of the affinity particle according to this embodiment with the specimen is preferably detected by a fluorescence depolarization method. Specifically, the method includes the steps of: mixing a test reagent with a specimen to provide a mixed liquid; irradiating the mixed liquid with polarized light; and separately detecting polarized light components of luminescence of the affinity particle in the mixed liquid.

Through optical detection of the above-mentioned aggregation reaction occurring in the mixed liquid, the target substance in the specimen is detected, and the concentration of the target substance can be measured.

### [Examples]

The present invention is specifically described below by way of Examples. However, the present invention is not limited to these Examples.

### (1) Production of Particles 1 to 12

Polyvinylpyrrolidone (PVP-K30: manufactured by Tokyo Chemical Industry Co., Ltd.) was dissolved in a 2-morpholinoethanesulfonic acid (MES) buffer solution (manufactured by Kishida Chemical Co., Ltd.) having a pH of 7 to prepare a solvent A.

[Tris(2-thenoyltrifluoroacetone)(bis(triphenylphosphineoxide))europium(III)] (manufactured by Central Techno Corporation, hereinafter abbreviated as "Eu(TTA)₃(TPPO)₂"), [tris(2-thenoyltrifluoroacetone)(triphenylphosphineoxide)(dibenzylsulfoxide)europium(II I)] (manufactured by Central Techno Corporation, hereinafter abbreviated as "Eu(TTA)₃(TPPO)(DBSO)"), or [tris(2-thenoyltrifluoroacetone)(phenanthroline)europium(III)] (manufactured by Central Techno Corporation, hereinafter abbreviated as "Eu(TTA)₃Phen") serving as a europium complex, a styrene monomer (manufactured by Kishida Chemical Co., Ltd.), and 3-methacryloxypropyltrimethoxysilane (manufactured by Tokyo Chemical Industry Co., Ltd., hereinafter abbreviated as "MPS") were mixed to prepare a reaction liquid B.

The reaction liquid B was added into a four-necked flask containing the solvent A, and the mixture was stirred with a mechanical stirrer set to 300 rpm. After 15 minutes of stirring under a nitrogen flow condition, the temperature of an oil bath that had been prepared was set to 70°C, and the nitrogen flow was performed for an additional 15 minutes. After the mixed liquid had been heated and stirred, an aqueous solution having dissolved therein potassium persulfate (hereinafter abbreviated as "KPS") (manufactured by Sigma-Aldrich) or ammonium persulfate (hereinafter abbreviated as "APS") (manufactured by Kishida Chemical Co., Ltd.) was added into the reaction solution, and emulsion polymerization was performed for 20 hours. After the polymerization reaction, the resultant suspension was subjected to ultrafiltration with about 4 L of ion-exchanged water through use of an ultrafiltration membrane having a molecular weight cutoff of 100K so that the product was washed. Thus, dispersions of particles 1 to 12 were obtained. The mass ratios of the respective reagents used in the production of the particles 1 to 12 are shown in Table 1.

In the production of the particles 9, ethanol was added to the solvent A at a mass ratio of 0.1.

**[Table 1]**

| Sample | Solvent A | | |
|---|---|---|---|
| | Solvent amount | MES concentration | PVP-K30 |
| Particles 1 | 180 | 40 | 0.7 |
| Particles 2 | 80 | 100 | 0.7 |
| Particles 3 | 80 | 100 | 0.7 |
| Particles 4 | 80 | 100 | 0.7 |
| Particles 5 | 180 | 40 | 0.7 |
| Particles 6 | 80 | 100 | 0.7 |
| Particles 7 | 180 | 40 | 0.7 |
| Particles 8 | 80 | 50 | 0.7 |
| Particles 9 | 80 | 100 | 0.7 |
| Particles 10 | 80 | 100 | 0.7 |
| Particles 11 | 80 | 100 | 0.7 |
| Particles 12 | 150 | 100 | 0.7 |

| Sample | Reaction liquid B | | | | | Catalyst | |
|---|---|---|---|---|---|---|---|
| | Eu(TTA) ₃(TPPO)₂ | Eu(TTA)₃( TPPO)(DB SO) | Eu(TTA)₃ Phen | Styrene monomer | MPS | KPS | APS |
| Particles 1 | 0.12 | | | 3.2 | 1.0 | 0.5 | |
| Particles 2 | 0.12 | | | 3.5 | 1.0 | 0.5 | |
| Particles 3 | 0.12 | | | 3.2 | 1.0 | 0.15 | |
| Particles 4 | 0.08 | | | 4.2 | 1.0 | 0.1 | |
| Particles 5 | 0.12 | | | 3.2 | 1.0 | 0.15 | |
| Particles 6 | 0.04 | | | 3.2 | 1.0 | 0.05 | |
| Particles 7 | | 0.12 | | 3.2 | 1.0 | 0.5 | |
| Particles 8 | | | 0.03 | 3.2 | 1.0 | 0.05 | |
| Particles 9 | | | 0.06 | 3.2 | 1.0 | 0.15 | |
| Particles 10 | | | 0.03 | 3.2 | 1.0 | 0.1 | |
| Particles 11 | | | 0.06 | 3.2 | 1.0 | 0.1 | |
| Particles 12 | 0.16 | | | 3.2 | 1.0 | | 0.4 |

An aliquot of each of the dispersions of the particles 1 to 12 obtained by the emulsion polymerization was taken and added to an aqueous solution having dissolved therein 1 mass% of Tween 20 (manufactured by Kishida Chemical Co., Ltd.). After 10 minutes of stirring, a silane coupling agent, X12-1135 (manufactured by Shin-Etsu Chemical Co., Ltd.), was added, and the mixture was stirred overnight. After the stirring, the dispersion was centrifuged, the supernatant was removed, and the precipitate was redispersed with pure water. The operations of centrifugation and redispersion were performed 3 or more times to wash the product. The precipitate after the washing was redispersed in pure water. Thus, ligand-bonding functional groups were introduced into the particles 1 to 8. A mass ratio among the particles, pure water, and X12-1135 loaded was set to 1:300:2.

### (Example 1)

A particle dispersion in which a sample corresponding to the synthesized particles 1 was diluted with pure water to a concentration of 0.002 mg/mL was prepared.

### (Example 2)

A particle dispersion in which a sample corresponding to the synthesized particles 2 was diluted with pure water to a concentration of 0.002 mg/mL was prepared.

### (Example 3)

A particle dispersion in which a sample corresponding to the synthesized particles 3 was diluted with pure water to a concentration of 0.002 mg/mL was prepared.

### (Example 4)

A particle dispersion in which a sample corresponding to the synthesized particles 4 was diluted with pure water to a concentration of 0.002 mg/mL was prepared.

### (Example 5)

A particle dispersion in which a sample corresponding to the synthesized particles 5 was diluted with pure water to a concentration of 0.002 mg/mL was prepared.

### (Example 6)

A particle dispersion in which a sample corresponding to the synthesized particles 6 was diluted with pure water to a concentration of 0.002 mg/mL was prepared.

### (Example 7)

A particle dispersion in which a sample corresponding to the synthesized particles 7 was diluted with pure water to a concentration of 0.002 mg/mL was prepared.

### (Example 8)

A particle dispersion in which a sample corresponding to the synthesized particles 12 was diluted with pure water to a concentration of 0.002 mg/mL was prepared.

### (Production of CRP Antibody-modified Affinity Particles)

An aliquot of 0.25 mL of the particle dispersion at 1.2 wt% corresponding to the synthesized particles 3 was taken, and the solvent was replaced by 1.6 mL of a MES buffer solution having a pH of 6.0. To the particle MES buffer solution, 1-[3-(dimethylamino)propyl]-3-ethylcarbodiimide and N-hydroxysulfosuccinimide sodium were added at 0.5 wt%, and the mixture was subjected to a reaction at 25°C for 1 hour. After the reaction, the dispersion was washed with a MES buffer solution having a pH of 5.0, an anti-CRP antibody was added at 100 µg/mL, and the anti-CRP antibody was bonded to the particles at 25°C for 2 hours. After the bonding, the particles were washed with a Tris buffer solution having a pH of 8. After the reaction, the particles were washed with a phosphate buffer solution to provide CRP antibody-modified affinity particles 3 having a concentration of 0.3 wt% (sometimes referred to as "affinity particles 3").

The bonding of the antibody to the particles was recognized by measuring the amount of a reduction in antibody concentration in the buffer solution having added thereto the antibody by BCA assay.

A change in fluorescence polarization was observed before and after the obtained affinity particles 3 were mixed with a CRP antigen diluted with a MES buffer solution. An investigation was performed with the affinity particles 3 fixed at 0.0001 mg/mL, and with CRP at an antigen concentration of from 0 pg/mL to 1,000 pg/mL. The observation was performed at normal temperature. A measurement method for fluorescence polarization is described later.

### (Comparative Example 1)

A particle dispersion in which a sample corresponding to the synthesized particles 8 was diluted with pure water to a concentration of 0.002 mg/mL was prepared.

### (Comparative Example 2)

A particle dispersion in which a sample corresponding to the synthesized particles 9 was diluted with pure water to a concentration of 0.002 mg/mL was prepared.

### (Comparative Example 3)

A particle dispersion in which a sample corresponding to the synthesized particles 10 was diluted with pure water to a concentration of 0.002 mg/mL was prepared.

### (Comparative Example 4)

A particle dispersion in which a sample corresponding to the synthesized particles 11 was diluted with pure water to a concentration of 0.002 mg/mL was prepared.

### (Evaluations of Products)

The products in Examples and Comparative Examples were each subjected to such evaluations as described below.

The shapes of the products were evaluated with an electron microscope (S-5500 manufactured by Hitachi High-Technologies Corporation).

The average particle diameters of the products were evaluated by using dynamic light scattering (Zetasizer Nano S manufactured by Malvern).

The concentration of a suspension having each of the products dispersed therein was evaluated with a mass spectrometer (Thermo plus TG8120 manufactured by Rigaku Corporation).

A fluorescence spectrum of each of the products was measured with excitation light at 340 nm and with a polarizer placed in an optical path on each of an excitation side and a luminescence side. The measurement was performed with the polarizer on the excitation side being fixed, and the polarizer on the luminescence side being placed in a direction parallel or perpendicular to the polarizer on the excitation side. An apparatus used was a fluorescence spectrophotometer F-4500 (manufactured by Hitachi High-Tech Science Corporation). The peak wavelength of observation light for analyzing polarized luminescence was set to 611 nm. The resultant data on polarized luminescence was analyzed with the equation (8) described above to determine the polarization anisotropy "r".

The evaluation of an antigen-antibody reaction using CRP was performed with a microplate reader. Nivo (manufactured by PerkinElmer Co., Ltd.) was used as a device. A filter having a central wavelength of 355 nm and a half width of 40 nm was used on an excitation light side, and a filter having a central wavelength of 615 nm and a half width of 8 nm was used on a luminescence side. D400 was used as a dichroic mirror. A measurement time was set to 1,000 milliseconds, and a change in polarization anisotropy was observed over 30 minutes from the start of the reaction. A measurement temperature was fixed to 37°C.

Nonspecific agglutination suppression evaluation of each of the products was performed as described below.

60 µl of a human serum solution diluted 15-fold with a buffer solution was added to each particle dispersion (3 mg/mL) produced by the same method as those of Examples 1 to 5 and Comparative Examples 1 to 3, and the mixture was kept at 37°C for 5 minutes. Absorbances at 527 nm were measured before and after the temperature keeping, and the amount of change in absorbance before and after the temperature keeping was measured 3 times. Table 2 shows the average value of the 3 times. Evaluation was performed as follows: when the amount of change in "absorbance×10,000" value was less than 1,000, it was determined that nonspecific agglutination was suppressed, and when the amount was 1,000 or more, it was determined that nonspecific agglutination occurred.

### (Performance Evaluation)

According to the results of the nonspecific agglutination suppression evaluation, in each of Examples 1 to 8 and Comparative Examples 1 to 4, the change in absorbance was equal to or less than the specific numerical value (the amount of change in "absorbance×10,000" value was 1,000 or less), and hence it was recognized that the particles were capable of suppressing nonspecific adsorption.

The structures and physical properties of the particle materials of Examples 1 to 8 and Comparative Examples 1 to 4 are shown in Table 2 (the particle diameter in Table 2 shows an average particle diameter of the particles).

**[Table 2]**

| | Example | Example | Example | Example | Example | Example |
|---|---|---|---|---|---|---|
| | 1 | 2 | 3 | 4 | 5 | 6 |
| Particle diameter/nm | 89 | 95 | 125 | 163 | 155 | 156 |
| Eu complex | Eu(TTA)₃(TPPO)₂ | | | | | |
| Eu concentration/ppm | 2,424 | 2,108 | 1,978 | 1,952 | 1,411 | 1,170 |
| Luminescence intensity/cps | 3,132 | 3,175 | 1,221 | 2,070 | 1,656 | 1,212 |
| Anisotropy "r" | 0.0549 | 0.0566 | 0.0719 | 0.0813 | 0.0662 | 0.063 |
| Absorbance×10,000 | 50 | 70 | 10 | 30 | 30 | 40 |

| | Example | Example | Comparative Example | Comparative Example | Comparative Example | Comparative Example |
|---|---|---|---|---|---|---|
| | 7 | 8 | 1 | 2 | 3 | 4 |
| Particle diameter/nm | 109 | 115 | 136 | 126 | 106 | 98 |
| Eu complex | Eu(TTA)₃(TP PO)(DBSO) | Eu(TTA) ₃(TPPO)₂ | Eu(TTA)₃Phen | | | |
| Eu concentration/ppm | 2,209 | 3,895 | 864 | 403 | 757 | 974 |
| Luminescence intensity/cps | 1,877 | 3,585 | 1,099 | 212 | 836 | 997 |
| Anisotropy "r" | 0.0641 | 0.068 | - | - | - | - |
| Absorbance×10,000 | 60 | 70 | 40 | 30 | 20 | 20 |

The particles of Examples 1, 2, 3, 4, 5, 6, 7, and 8 had diameters (average particle diameters) of 89 nm, 95 nm, 125 nm, 163 nm, 155 nm, 156 nm, 109 nm, and 115 nm, respectively. The particles of Examples 1, 2, 3, 4, 5, 6, 7, and 8 had luminescence intensities of 3,132 cps, 3,175 cps, 1,221 cps, 2,070 cps, 1,656 cps, 1,212 cps, 1,877 cps, and 3,585 cps, respectively. In addition, the particles of Examples 1, 2, 3, 4, 5, 6, 7, and 8 had amounts of a europium element present therein of 2,424 ppm, 2,108 ppm, 1,978 ppm, 1,952 ppm, 1,411 ppm, 1,170 ppm, 2,209 ppm, and 3,895 ppm, respectively. For the particles each having a high europium concentration around 2,000 ppm out of the particles of Examples 1, 2, 3, 4, 5, 6, 7, and 8, a relationship between values of polarization anisotropy "r" of the particles determined from the results of polarization measurement and their particle sizes is shown in FIG. 2.

It was recognized from FIG. 2 that the particle size and the polarization anisotropy had an extremely high correlation.

From Table 2, none of the particles of Comparative Examples 1, 2, 3, and 4 had a europium concentration of more than 1,000 ppm, and their luminescence intensities were 1,099 cps, 212 cps, 836 cps, and 997 cps, respectively, which fell short of the numerical values of Examples. A relationship between the europium concentrations in the particles and the luminescence intensities thereof is shown in FIG. 3. In FIG. 3, black circles represent the results of Examples, and black triangles represent the results of Comparative Examples. It was able to be recognized from FIG. 3 that as the europium concentration in the particles became higher, the luminescence intensity was increased more. In addition, all the particles of Examples exhibited higher luminescence intensities than those of the particles of Comparative Examples. It was also recognized that the europium complexes used in the particles of Comparative Examples each had a low solubility in the particle substrate, and their concentrations were not able to be increased any more.

Meanwhile, when a europium complex having such high solubility that the europium complex was admixed with the particle substrate was used, particles themselves were not able to be synthesized. The particles exhibiting strong luminescence were able to be synthesized only when the complexes of Examples of the present invention were used.

The results of quantification of the antigen-antibody reaction using the CRP antibody, which was evaluated for the affinity particles 3 produced in the "Production of CRP Antibody-modified Affinity Particles" section, through use of a fluorescence depolarization method are shown in Table 3.

**[Table 3]**

| | Affinity particles 3 | | | | | |
|---|---|---|---|---|---|---|
| Particle diameter/nm | 125 | | | | | |
| CRP antigen concentration pg/mL | 1 | 5 | 10 | 100 | 1,000 | 10,000 |
| Change in anisotropy/slope of amount of change Δr over 30 minutes of measurement | 0.00414 | 0.00442 | 0.00517 | 0.00603 | 0.00637 | 0.0102 |

It was recognized from Table 3 that when the slope of a change Δr in degree of polarization, which was obtained by dividing the amount of change in polarization anisotropy over 30 minutes by the time, was determined, the amount of change in polarization anisotropy before and after the antigen-antibody reaction changed in accordance with the CRP antibody concentration. The CRP antigen concentrations used are extremely small and in a region in which detection cannot be performed by a general latex agglutination method (ng/mL order). For particles having BSA supported thereon, the numerical value of the polarization anisotropy deviates from prediction owing to a broad particle size distribution, and hence, in the case of detecting a target substance at a low concentration, it is difficult to accurately measure a change ratio. In addition, it was recognized that, even under the state of having a low concentration of 0.001 mg/ml, the particles exhibited sufficiently strong luminescence to the extent that the luminescence was able to be detected and a change in polarization anisotropy was able to be grasped.

Thus, it was revealed that the particles according to Examples of the present invention were polarized luminescent materials each having high accuracy.

Accordingly, the particles according to Examples of the present invention can be used to provide particles for a specimen test for a fluorescence depolarization method having extremely high detection sensitivity. In particular, the particles according to Examples of the present invention each have a high luminescence intensity, and are hence each excellent in reducing effect on the detectable lower limit of the polarization anisotropy. Accordingly, the particles according to Examples of the present invention are each suitable for detection of a target substance at a low concentration.

According to the particles of this embodiment, a change in anisotropy of polarized luminescence can be detected with high sensitivity in response to the aggregation/dispersion behavior of the particles. That is, the particles of this embodiment are suitably used for a fluorescence depolarization method. According to the particles of this embodiment, a target substance can be detected and quantified with high sensitivity by the fluorescence depolarization method.

In addition, when a hydrophilic layer is formed on the surfaces of the particles, nonspecific adsorption to the particles of this embodiment is effectively suppressed, and hence there is no need to use a nonspecific adsorbent such as BSA. Accordingly, the target substance can be detected with high sensitivity through use of the particles of this embodiment based on the fluorescence depolarization method.

The present invention is not limited to the embodiments described above, and various changes and modifications may be made without departing from the scope of the present invention as defined by the appended claims.

The present application claims priority based on Japanese Patent Application No. 2021-192077 filed on November 26, 2021, and Japanese Patent Application No. 2022-185946 filed on November 21, 2022.

### [Reference Signs List]

1 particle substrate
2 hydrophilic layer
3 europium complex

## Claims

1. A particle comprising a particle substrate,
wherein the particle substrate includes: a polymer containing a styrene unit and an organic silane unit; and a europium complex represented by the following formula (1), and
wherein the particle substrate contains a siloxane bond on at least a surface of the particle substrate:
Eu(A)ₓ(B)_{y}(C)_{z} ··· (1)
in the formula (1), (A) represents a ligand represented by the following formula (2), (B) represents a ligand represented by the following formula (3) or (4), and (C) represents a ligand represented by the following formula (5): 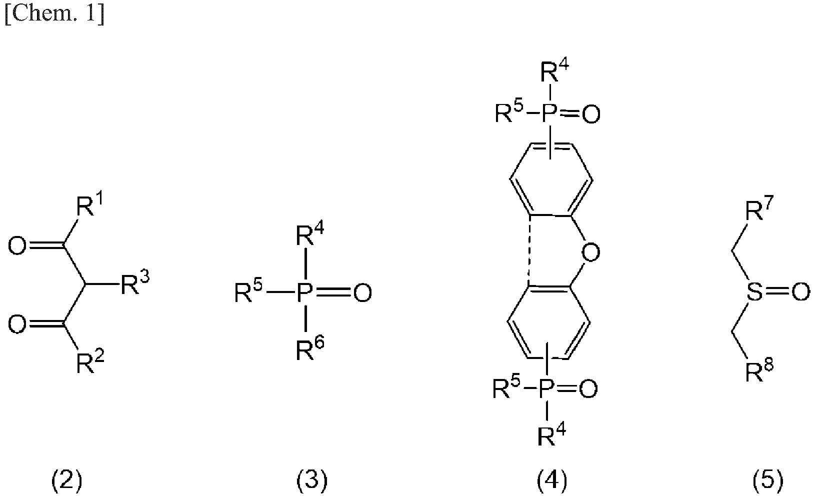 in the formulae (2) to (5), R¹ and R² each independently represent an alkyl group, a perfluoroalkyl group, a phenyl group, or a thiophene group, and the groups may each have a substituent, R³ represents a hydrogen atom or a methyl group, R⁴ and R⁵ each independently represent an alkyl group or a phenyl group, and the groups may each have a substituent, R⁶ represents an alkyl group, a phenyl group, or a triphenylene group, and the groups may each have a substituent, and R⁷ and R⁸ each independently represent an alkyl group or a phenyl group, and the groups may each have a substituent; in the formula (4), a bond represented by a broken line may or may not be present; the substituents are each independently any one of a methyl group, a fluoro group, a chloro group, or a bromo group; the alkyl groups each independently have 2 or more and 12 or less carbon atoms; and x, y, and z satisfy the following equations: $x = 3 ;$ $y = 1 or 2 ;$ $z = 0 or 1 ;$ and $x + y + z = 4 or 5 .$

2. The particle according to claim 1, wherein the particle has a polarization anisotropy <r> of 0.01 or more, which is determined by the following equation (9):
[Math. 1] $\begin{matrix}\left〈r\right〉 = \frac{{I}_{VV} - G ∗ {I}_{V H}}{{I}_{VV} + 2 ∗ G ∗ {I}_{VH}} \\ G = \frac{{I}_{HV}}{{I}_{HH}}\end{matrix}$ in the equation (9),
<r> represents the polarization anisotropy,
Ivv represents a luminescence intensity of a luminescence component having a vibration direction parallel to that of a first polarized light beam at a time of excitation by the first polarized light beam,
I_{VH} represents a luminescence intensity of a luminescence component having a vibration direction orthogonal to that of the first polarized light beam at the time of excitation by the first polarized light beam,
I_{HV} represents a luminescence intensity of a luminescence component having a vibration direction orthogonal to that of a second polarized light beam having a vibration direction orthogonal to that of the first polarized light beam at a time of excitation by the second polarized light beam, and
I_{HH} represents a luminescence intensity of a luminescence component having a vibration direction parallel to that of the second polarized light beam having a vibration direction orthogonal to that of the first polarized light beam at the time of excitation by the second polarized light beam.

3. The particle according to claim 1 or 2, wherein the particle includes a layer containing a hydrophilic polymer on an outside of the particle substrate.

4. The particle according to claim 3, wherein the hydrophilic polymer is polyvinylpyrrolidone.

5. The particle according to any one of claims 1 to 4,
wherein the particle has a ligand-bonding functional group capable of bonding a ligand to an outside of the particle substrate, and
wherein the ligand-bonding functional group has at least one functional group selected from the group consisting of: a carboxy group; an amino group; a thiol group; an epoxy group; a maleimide group; and a succinimidyl group.

6. The particle according to any one of claims 1 to 5, wherein the particle has an average particle diameter of 50 nm or more and 300 nm or less.

7. The particle according to any one of claims 1 to 6, wherein the particle satisfies the following formula: $A 2 - A 1 \leq 0.1$ where, with regard to a mixture obtained by adding 30 µL of a 0.1 wt% dispersion of the particle to 60 µL of a buffer solution mixed with 16 µL of human serum diluted 15-fold, an absorbance of the mixture immediately after the addition is represented by A1, and an absorbance of the mixture after being left to stand at 37°C for 5 minutes after the addition is represented by A2, provided that the absorbances are measured at an optical path of 10 mm and a wavelength of 572 nm.

8. A method of producing a particle comprising:
a first step of mixing radically polymerizable monomers including styrene and a radically polymerizable organic silane, a radical polymerization initiator, a europium complex represented by the following formula (1), a hydrophilic polymer, and an aqueous medium to prepare an emulsion; and
a second step of polymerizing the radically polymerizable monomers by stirring the emulsion:
Eu(A)ₓ(B)_{y}(C)_{z} ··· (1)
in the formula (1), (A) represents a ligand represented by the following formula (2), (B) represents a ligand represented by the following formula (3) or (4), and (C) represents a ligand represented by the following formula (5): 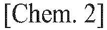 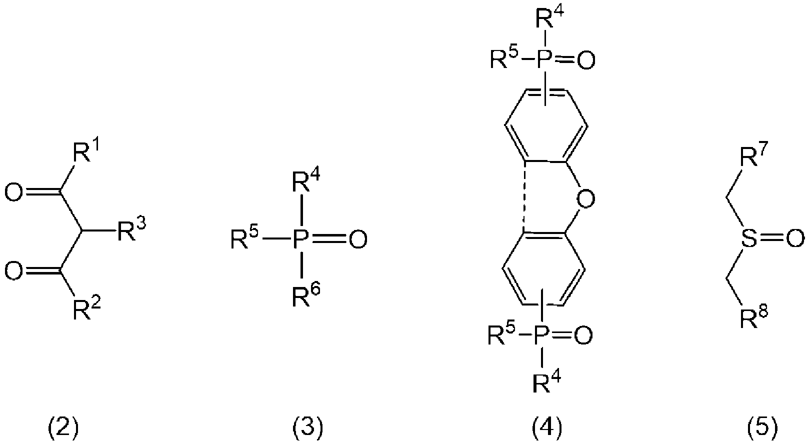 in the formulae (2) to (5), R¹ and R² each independently represent an alkyl group, a perfluoroalkyl group, a phenyl group, or a thiophene group, and the groups may each have a substituent, R³ represents a hydrogen atom or a methyl group, R⁴ and R⁵ each independently represent an alkyl group or a phenyl group, and the groups may each have a substituent, R⁶ represents an alkyl group, a phenyl group, or a triphenylene group, and the groups may each have a substituent, and R⁷ and R⁸ each independently represent an alkyl group or a phenyl group and the groups may each have a substituent; in the formula (4), a bond represented by a broken line may or may not be present; the substituents are each independently any one of a methyl group, a fluoro group, a chloro group, or a bromo group; the alkyl groups each independently have 2 or more and 12 or less carbon atoms; and x, y, and z satisfy the following equations: $x = 3 ;$ $y = 1 or 2 ;$ $z = 0 or 1 ;$ and $x + y + z = 4 or 5 .$

9. A method of producing a test reagent comprising a step of dispersing the particle of claim 1 in a dispersion medium.

10. The method of producing a test reagent according to claim 9, wherein the particle has an average particle diameter of 50 nm or more and 300 nm or less.

11. An affinity particle comprising:
the particle of claim 5; and
a ligand bonded to the ligand-bonding functional group.

12. The affinity particle according to claim 11, wherein the ligand contains one selected from the group consisting of: an antibody; an antigen; a protein; and a nucleic acid.

13. A test reagent for *in vitro* diagnosis comprising:
the affinity particle of claim 11 or 12; and
a dispersion medium for dispersing the affinity particle.

14. The test reagent for *in vitro* diagnosis according to claim 13, wherein the test reagent is used for detection of an antigen or an antibody in a specimen by an agglutination method.

15. A test kit for *in vitro* diagnosis comprising:
the test reagent for *in vitro* diagnosis of claim 13 or 14; and
a case enclosing the test reagent.

16. A method of detecting a target substance in a specimen, the method comprising a step of mixing the test reagent for *in vitro* diagnosis of claim 13 or 14 with the specimen.

17. A method of detecting a target substance in a specimen by a fluorescence depolarization method, the detection method comprising the steps of:
mixing the test reagent for *in vitro* diagnosis of claim 13 or 14 with the specimen to provide a mixed liquid;
irradiating the mixed liquid with polarized light; and
detecting polarized light that is emitted by being excited by the polarized light with which the mixed liquid has been irradiated.

## Patentansprüche

1. Partikel, umfassend ein Partikelsubstrat,
wobei das Partikelsubstrat enthält: ein Polymer, enthaltend eine Styrol-Einheit und eine Organosilan-Einheit; und einen Europiumkomplex, der durch die folgende Formel (1) dargestellt ist, und
wobei das Partikelsubstrat eine Siloxanbindung auf mindestens einer Oberfläche des Partikelsubstrats enthält:
Eu(A)ₓ(B)_{y}(C)_{z} ··· (1)
wobei in der Formel (1) (A) einen Liganden darstellt, der durch die folgende Formel (2) dargestellt ist, (B) einen Liganden darstellt, der durch die folgende Formel (3) oder (4) dargestellt ist, und (C) einen Liganden darstellt, der durch die folgende Formel (5) dargestellt ist: 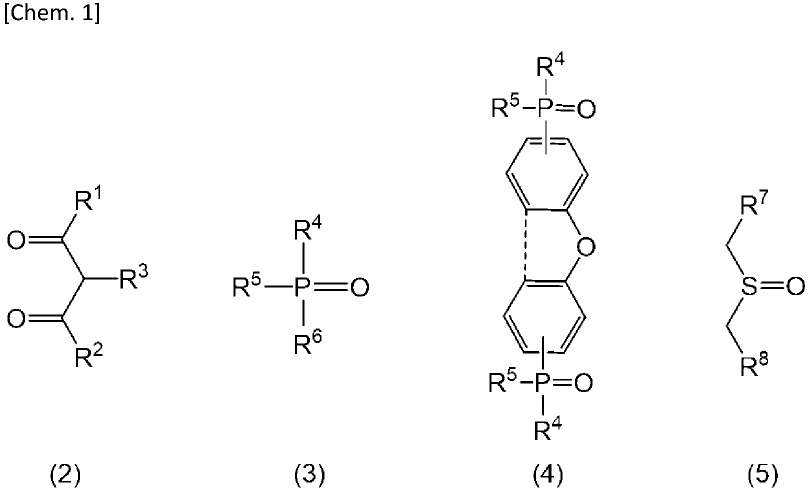 wobei in den Formeln (2) bis (5) R¹ und R² jeweils unabhängig eine Alkylgruppe, eine Perfluoralkylgruppe, eine Phenylgruppe oder eine Thiophengruppe darstellen, und die Gruppen jeweils einen Substituenten aufweisen können, R³ ein Wasserstoffatom oder eine Methylgruppe darstellt, R⁴ und R⁵ jeweils unabhängig eine Alkylgruppe oder eine Phenylgruppe darstellen, und die Gruppen jeweils einen Substituenten aufweisen können, R⁶ eine Alkylgruppe, eine Phenylgruppe oder eine Triphenylengruppe darstellt, und die Gruppen jeweils einen Substituenten aufweisen können, und R⁷ und R⁸ jeweils unabhängig eine Alkylgruppe oder eine Phenylgruppe darstellen, und die Gruppen jeweils einen Substituenten aufweisen können; wobei in der Formel (4) eine Bindung, die durch eine gestrichelte Linie dargestellt ist, vorhanden sein kann oder nicht vorhanden sein kann; wobei die Substituenten jeweils unabhängig eine von einer Methylgruppe, einer Fluorgruppe, einer Chlorgruppe oder einer Bromgruppe sind; wobei die Alkylgruppen jeweils unabhängig 2 oder mehr und 12 oder weniger Kohlenstoffatome aufweisen; und x, y und z die folgenden Gleichungen erfüllen: $\begin{matrix}x = 3 ; \\ y = 1 oder 2 ;\end{matrix}$ $z = 0 oder 1 ;$ und $x + y + z = 4 oder 5 .$

2. Partikel nach Anspruch 1, wobei das Partikel eine Polarisationsanisotropie <r> von 0,01 oder mehr aufweist, die durch die folgende Gleichung (9) bestimmt wird:
[Math. 1] $\begin{matrix}\left〈r\right〉 = \frac{{I}_{VV} - G ∗ {I}_{VH}}{{I}_{VV} + 2 ∗ G ∗ {I}_{VH}} \\ G = \frac{{I}_{HV}}{{I}_{HH}}\end{matrix}$ wobei in der Gleichung (9)
<r> die Polarisationsanisotropie darstellt,
I_{VV} eine Lumineszenzintensität einer Lumineszenzkomponente mit einer Schwingungsrichtung parallel zu der eines ersten polarisierten Lichtstrahls zu einem Zeitpunkt der Anregung durch den ersten polarisierten Lichtstrahl darstellt,
I_{VH} eine Lumineszenzintensität einer Lumineszenzkomponente mit einer Schwingungsrichtung orthogonal zu der des ersten polarisierten Lichtstrahls zu dem Zeitpunkt der Anregung durch den ersten polarisierten Lichtstrahl darstellt,
I_{HV} eine Lumineszenzintensität einer Lumineszenzkomponente mit einer Schwingungsrichtung orthogonal zu der eines zweiten polarisierten Lichtstrahls mit einer Schwingungsrichtung orthogonal zu der des ersten polarisierten Lichtstrahls zu einem Zeitpunkt der Anregung durch den zweiten polarisierten Lichtstrahl darstellt, und
I_{HH} eine Lumineszenzintensität einer Lumineszenzkomponente mit einer Schwingungsrichtung parallel zu der des zweiten polarisierten Lichtstrahls mit einer Schwingungsrichtung orthogonal zu der des ersten polarisierten Lichtstrahls zu dem Zeitpunkt der Anregung durch den zweiten polarisierten Lichtstrahl darstellt.

3. Partikel nach Anspruch 1 oder 2, wobei das Partikel eine Schicht enthält, die ein hydrophiles Polymer auf einer Außenseite des Partikelsubstrats enthält.

4. Partikel nach Anspruch 3, wobei das hydrophile Polymer Polyvinylpyrrolidon ist.

5. Partikel nach einem der Ansprüche 1 bis 4,
wobei das Partikel eine ligandenbindende funktionelle Gruppe aufweist, die in der Lage ist, einen Liganden an eine Außenseite des Partikelsubstrats zu binden, und
wobei die ligandenbindende funktionelle Gruppe mindestens eine funktionelle Gruppe aufweist, die ausgewählt ist aus der Gruppe bestehend aus: einer Carboxygruppe; einer Aminogruppe; einer Thiolgruppe; einer Epoxygruppe; einer Maleimidgruppe; und einer Succinimidylgruppe.

6. Partikel nach einem der Ansprüche 1 bis 5, wobei das Partikel einen durchschnittlichen Partikeldurchmesser von 50 nm oder mehr und 300 nm oder weniger aufweist.

7. Partikel nach einem der Ansprüche 1 bis 6, wobei das Partikel die folgende Formel erfüllt: $A 2 - A 1 \leq 0,1$ wobei in Bezug auf eine Mischung, erhalten durch Zugeben von 30 µL einer 0,1 Gew.-% Dispersion des Partikels zu 60 µL einer Pufferlösung, gemischt mit 16 µL 15-fach verdünntem humanem Serum, ein Absorptionsgrad der Mischung unmittelbar nach der Zugabe durch A1 dargestellt wird und ein Absorptionsgrad der Mischung nach Stehenlassen bei 37 °C für 5 Minuten nach der Zugabe durch A2 dargestellt wird, vorausgesetzt, dass die Absorptionsgrade bei einem optischen Weg von 10 mm und einer Wellenlänge von 572 nm gemessen werden.

8. Verfahren zur Herstellung eines Partikels, umfassend:
einen ersten Schritt des Mischens von radikalisch polymerisierbaren Monomeren, enthaltend Styrol und ein radikalisch polymerisierbares Organosilan, einen radikalischen Polymerisationsinitiator, einen Europiumkomplex, der durch die folgende Formel (1) dargestellt ist, ein hydrophiles Polymer und ein wässriges Medium, um eine Emulsion herzustellen; und
einen zweiten Schritt des Polymerisierens der radikalisch polymerisierbaren Monomere durch Rühren der Emulsion:
Eu(A)ₓ(B)_{y}(C)_{z} ··· (1)
wobei in der Formel (1) (A) einen Liganden darstellt, der durch die folgende Formel (2) dargestellt ist, (B) einen Liganden darstellt, der durch die folgende Formel (3) oder (4) dargestellt ist, und (C) einen Liganden darstellt, der durch die folgende Formel (5) dargestellt ist: 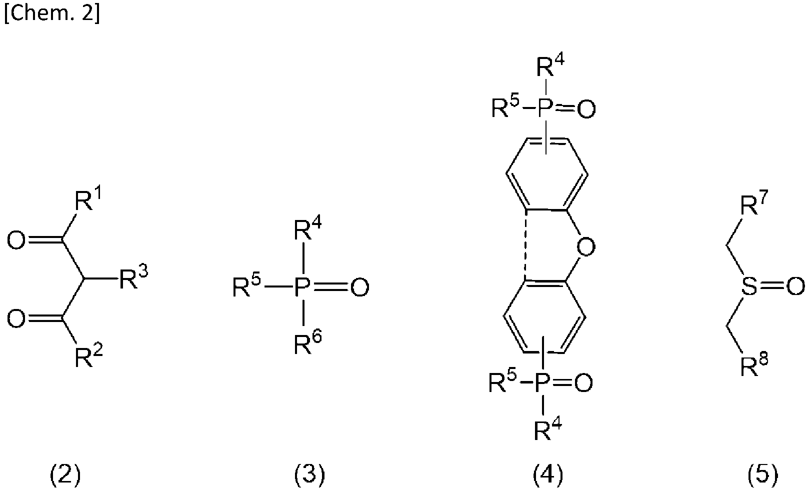 wobei in den Formeln (2) bis (5) R¹ und R² jeweils unabhängig eine Alkylgruppe, eine Perfluoralkylgruppe, eine Phenylgruppe oder eine Thiophengruppe darstellen, und die Gruppen jeweils einen Substituenten aufweisen können, R³ ein Wasserstoffatom oder eine Methylgruppe darstellt, R⁴ und R⁵ jeweils unabhängig eine Alkylgruppe oder eine Phenylgruppe darstellen, und die Gruppen jeweils einen Substituenten aufweisen können, R⁶ eine Alkylgruppe, eine Phenylgruppe oder eine Triphenylengruppe darstellt, und die Gruppen jeweils einen Substituenten aufweisen können, und R⁷ und R⁸ jeweils unabhängig eine Alkylgruppe oder eine Phenylgruppe darstellen, und die Gruppen jeweils einen Substituenten aufweisen können; wobei in der Formel (4) eine Bindung, die durch eine gestrichelte Linie dargestellt ist, vorhanden sein kann oder nicht vorhanden sein kann; wobei die Substituenten jeweils unabhängig eine von einer Methylgruppe, einer Fluorgruppe, einer Chlorgruppe oder einer Bromgruppe sind; wobei die Alkylgruppen jeweils unabhängig 2 oder mehr und 12 oder weniger Kohlenstoffatome aufweisen; und x, y und z die folgenden Gleichungen erfüllen: $x = 3 ;$ $y = 1 oder 2 ;$ $z = 0 oder 1 ;$ und $x + y + z = 4 oder 5 .$

9. Verfahren zur Herstellung eines Testreagens, umfassend einen Schritt des Dispergierens des Partikels nach Anspruch 1 in einem Dispersionsmedium.

10. Verfahren zur Herstellung eines Testreagens nach Anspruch 9, wobei das Partikel einen durchschnittlichen Partikeldurchmesser von 50 nm oder mehr und 300 nm oder weniger aufweist.

11. Affinitätspartikel, umfassend:
das Partikel nach Anspruch 5; und
einen Liganden, der an die ligandenbindende funktionelle Gruppe gebunden ist.

12. Affinitätspartikel nach Anspruch 11, wobei der Ligand einen enthält, der ausgewählt ist aus der Gruppe bestehend aus: einem Antikörper; einem Antigen; einem Protein; und einer Nukleinsäure.

13. Testreagens zur *in vitro*-Diagnose, umfassend:
das Affinitätspartikel nach Anspruch 11 oder 12; und
ein Dispersionsmedium zum Dispergieren des Affinitätspartikels.

14. Testreagens zur *in vitro*-Diagnose nach Anspruch 13, wobei das Testreagens zum Nachweis eines Antigens oder eines Antikörpers in einer Probe durch ein Agglutinationsverfahren verwendet wird.

15. Testkit zur *in vitro*-Diagnose, umfassend:
das Testreagens zur *in vitro*-Diagnose nach Anspruch 13 oder 14; und
ein Gehäuse, das das Testreagens umschließt.

16. Verfahren zum Nachweis einer Zielsubstanz in einer Probe, wobei das Verfahren einen Schritt des Mischens des Testreagens zur *in vitro*-Diagnose nach Anspruch 13 oder 14 mit der Probe umfasst.

17. Verfahren zum Nachweis einer Zielsubstanz in einer Probe durch ein Fluoreszenz-Depolarisationsverfahren, wobei das Nachweisverfahren die folgenden Schritte umfasst:
Mischen des Testreagens zur *in vitro*-Diagnose nach Anspruch 13 oder 14 mit der Probe, um eine gemischte Flüssigkeit bereitzustellen;
Bestrahlen der gemischten Flüssigkeit mit polarisiertem Licht; und
Nachweisen von polarisiertem Licht, das emittiert wird, indem es durch das polarisierte Licht, mit dem die gemischte Flüssigkeit bestrahlt wurde, angeregt wird.

## Revendications

1. Particule comprenant un substrat particulaire,
dans laquelle le substrat particulaire inclut : un polymère contenant une unité styrène et une unité silane organique ; et un complexe d'europium représenté par la formule (1) suivante, et
dans laquelle le substrat particulaire contient une liaison siloxane sur au moins une surface du substrat particulaire :
Eu(A)ₓ(B)_{y}(C)_{z} ... (1)
dans la formule (1), (A) représente un ligand représenté par la formule (2) suivante, (B) représente un ligand représenté par la formule (3) ou (4) suivante, et (C) représente un ligand représenté par la formule (5) suivante : 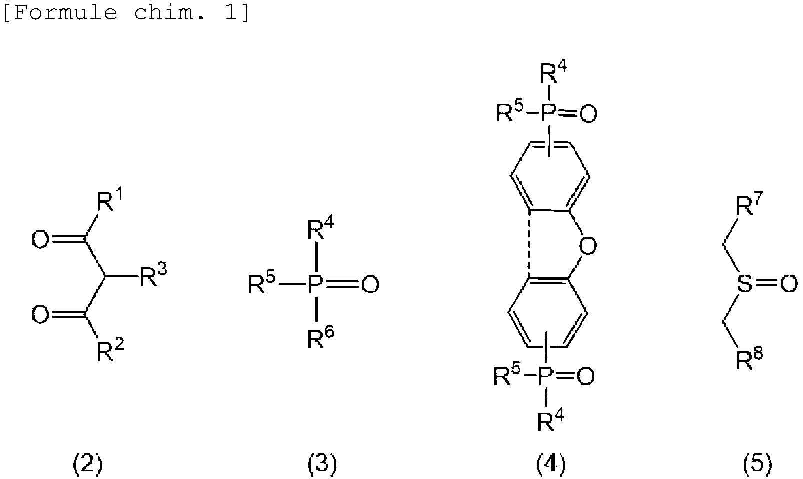
dans les formules (2) à (5), R¹ et R² représentent chacun indépendamment un groupe alkyle, un groupe perfluoroalkyle, un groupe phényle, ou un groupe thiophène, et les groupes peuvent chacun posséder un substituant, R³ représente un atome d'hydrogène ou un groupe méthyle, R⁴ et R⁵ représentent chacun indépendamment un groupe alkyle ou un groupe phényle, et les groupes peuvent chacun posséder un substituant, R⁶ représente un groupe alkyle, un groupe phényle ou un groupe triphénylène, et les groupes peuvent chacun posséder un substituant, et R⁷ et R⁸ représentent chacun indépendamment un groupe alkyle ou un groupe phényle, et les groupes peuvent chacun posséder un substituant ; dans la formule (4), une liaison représentée par une ligne discontinue peut ou peut ne pas être présente ; les substituants sont chacun indépendamment l'un quelconque parmi un groupe méthyle, un groupe fluoro, un groupe chloro ou un groupe bromo ; les groupes alkyle possèdent chacun indépendamment 2 atomes de carbone ou plus et 12 atomes de carbone ou moins ; et x, y, et z satisfont les équations suivantes : $x = 3 ;$ $y = 1 ou 2 ;$ $z = 0 ou 1 ;$ et $x + y + z = 4 ou 5 .$

2. Particule selon la revendication 1, dans laquelle la particule possède une anisotropie de polarisation <r> de 0,01 ou plus, qui est déterminée par l'équation (9) suivante :
[Formule math. 1] $\begin{matrix}\left〈r\right〉 = \frac{{I}_{VV} - G * {I}_{VH}}{{I}_{VV} + 2 * G * {I}_{VH}} \\ G = \frac{{I}_{HV}}{{I}_{HH}}\end{matrix}$ dans l'équation (9),
<r> représente l'anisotropie de polarisation,
I_{VV} représente une intensité de luminescence d'une composante de luminescence ayant une direction de vibration parallèle à celle d'un premier faisceau de lumière polarisée à un instant d'excitation par le premier faisceau de lumière polarisée,
I_{VH} représente une intensité de luminescence d'une composante de luminescence ayant une direction de vibration orthogonale à celle du premier faisceau de lumière polarisée à l'instant d'excitation par le premier faisceau de lumière polarisée,
I_{HV} représente une intensité de luminescence d'une composante de luminescence ayant une direction de vibration orthogonale à celle d'un deuxième faisceau de lumière polarisée ayant une direction de vibration orthogonale à celle du premier faisceau de lumière polarisée à un instant d'excitation par le deuxième faisceau de lumière polarisée, et
I_{HH} représente une intensité de luminescence d'une composante de luminescence ayant une direction de vibration parallèle à celle du deuxième faisceau de lumière polarisée ayant une direction de vibration orthogonale à celle du premier faisceau de lumière polarisée à l'instant d'excitation par le deuxième faisceau de lumière polarisée.

3. Particule selon la revendication 1 ou 2, dans laquelle la particule inclut une couche contenant un polymère hydrophile sur un extérieur du substrat particulaire.

4. Particule selon la revendication 3, dans laquelle le polymère hydrophile est la polyvinylpyrrolidone.

5. Particule selon l'une quelconque des revendications 1 à 4,
dans laquelle la particule possède un groupe fonctionnel de liaison de ligand apte à lier un ligand à un extérieur du substrat particulaire, et
dans laquelle le groupe fonctionnel de liaison de ligand possède au moins un groupe fonctionnel sélectionné dans le groupe consistant en : un groupe carboxy ; un groupe amino ; un groupe thiol ; un groupe époxy ; un groupe maléimide ; et un groupe succinimidyle.

6. Particule selon l'une quelconque des revendications 1 à 5, dans laquelle la particule possède un diamètre particulaire moyen de 50 nm ou plus et de 300 nm ou moins.

7. Particule selon l'une quelconque des revendications 1 à 6, dans laquelle la particule satisfait la formule suivante : $A 2 - A 1 \leq 0 , 1$ où, en ce qui concerne un mélange obtenu par l'ajout de 30 µL d'une dispersion à 0,1 % en poids de la particule dans 60 µL d'une solution tampon mélangée avec 16 µL de sérum humain dilué 15 fois, une absorbance du mélange immédiatement après l'ajout est représentée par A1, et une absorbance du mélange après avoir reposé à 37 °C pendant 5 minutes après l'ajout est représentée par A2, à condition que les absorbances soient mesurées sur un chemin optique de 10 mm et à une longueur d'onde de 572 nm.

8. Procédé de production d'une particule comprenant :
une première étape de mélange de monomères aptes à une polymérisation radicalaire incluant du styrène et un silane organique apte à une polymérisation radicalaire, un initiateur de polymérisation radicalaire, un complexe d'europium représenté par la formule (1) suivante, un polymère hydrophile et un milieu aqueux pour préparer une émulsion ; et
une deuxième étape de polymérisation des monomères aptes à une polymérisation radicalaire par l'agitation de l'émulsion :
Eu(A)ₓ(B)_{y}(C)_{z} ... (1)
dans la formule (1), (A) représente un ligand représenté par la formule (2) suivante, (B) représente un ligand représenté par la formule (3) ou (4) suivante, et (C) représente un ligand représenté par la formule (5) suivante : 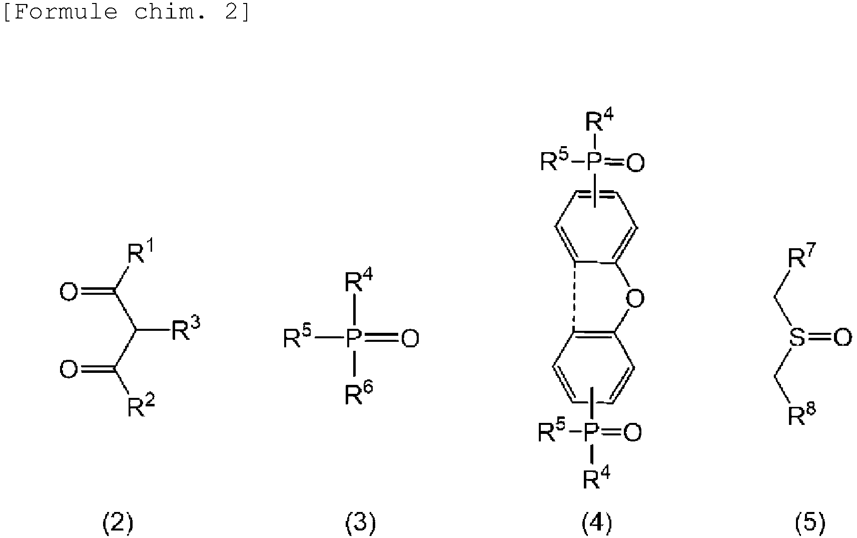
dans les formules (2) à (5), R¹ et R² représentent chacun indépendamment un groupe alkyle, un groupe perfluoroalkyle, un groupe phényle, ou un groupe thiophène, et les groupes peuvent chacun posséder un substituant, R³ représente un atome d'hydrogène ou un groupe méthyle, R⁴ et R⁵ représentent chacun indépendamment un groupe alkyle ou un groupe phényle, et les groupes peuvent chacun posséder un substituant, R⁶ représente un groupe alkyle, un groupe phényle ou un groupe triphénylène, et les groupes peuvent chacun posséder un substituant, et R⁷ et R⁸ représentent chacun indépendamment un groupe alkyle ou un groupe phényle et les groupes peuvent chacun posséder un substituant ;
dans la formule (4), une liaison représentée par une ligne discontinue peut ou peut ne pas être présente ; les substituants sont chacun indépendamment l'un quelconque parmi un groupe méthyle, un groupe fluoro, un groupe chloro ou un groupe bromo ; les groupes alkyle possèdent chacun indépendamment 2 atomes de carbone ou plus et 12 atomes de carbone ou moins ; et x, y, et z satisfont les équations suivantes : $x = 3 ;$ $y = 1 ou 2 ;$ $z = 0 ou 1 ;$ et $x + y + z = 4 ou 5 .$

9. Procédé de production d'un réactif de test comprenant une étape de dispersion de la particule selon la revendication 1 dans un milieu de dispersion.

10. Procédé de production d'un réactif de test selon la revendication 9, dans lequel la particule possède un diamètre particulaire moyen de 50 nm ou plus et de 300 nm ou moins.

11. Particule d'affinité comprenant :
la particule selon la revendication 5 ; et
un ligand lié au groupe fonctionnel de liaison de ligand.

12. Particule d'affinité selon la revendication 11, dans laquelle le ligand contient un élément sélectionné parmi le groupe consistant en : un anticorps ; un antigène ; une protéine ; et un acide nucléique.

13. Réactif de test pour diagnostic *in vitro* comprenant :
la particule d'affinité selon la revendication 11 ou 12 ; et
un milieu de dispersion pour la dispersion de la particule d'affinité.

14. Réactif de test pour diagnostic *in vitro* selon la revendication 13, dans lequel le réactif de test est utilisé pour la détection d'un antigène ou d'un anticorps dans un échantillon par un procédé d'agglutination.

15. Kit de test pour diagnostic *in vitro* comprenant :
le réactif de test pour diagnostic *in vitro* selon la revendication 13 ou 14 ; et
un étui renfermant le réactif de test.

16. Procédé de détection d'une substance cible dans un échantillon, le procédé comprenant une étape de mélange du réactif de test pour diagnostic *in vitro* selon la revendication 13 ou 14 avec l'échantillon.

17. Procédé de détection d'une substance cible dans un échantillon par un procédé de dépolarisation de fluorescence, le procédé de détection comprenant les étapes suivantes :
le mélange du réactif de test pour diagnostic *in vitro* selon la revendication 13 ou 14 avec l'échantillon pour fournir un liquide mélangé ;
l'irradiation du liquide mélangé avec une lumière polarisée ; et
la détection d'une lumière polarisée qui est émise en étant excitée par la lumière polarisée avec laquelle le liquide mélangé a été irradié.
